Europäisches Patentamt

European Patent Office

Office européen des brevets

(19)

(11) EP 0 212 670 B2

(12) **NEW EUROPEAN PATENT SPECIFICATION**

(45) Date of publication and mention
of the opposition decision:
**05.09.2001 Bulletin 2001/36**

(45) Mention of the grant of the patent:
**04.11.1992 Bulletin 1992/45**

(21) Application number: **86111928.7**

(22) Date of filing: **28.08.1986**

(51) Int Cl.[7]: **G01N 33/543**, G01N 33/542,
C12Q 1/00, C12Q 1/68,
G01N 33/566, G01N 33/68,
G01N 33/569, G01N 33/74,
G01N 33/88

(54) **Method for detecting an analyte moiety by means of signal localization**

Verfahren zum Nachweis eines Analytteiles mittels Signallokalisierung

Méthode de détection d'un élément d'un analyte par localisation d'un signal

(84) Designated Contracting States:
**AT BE CH DE FR GB IT LI LU NL SE**

(30) Priority: **29.08.1985 US 770828**
**15.05.1986 US 863742**
**09.09.1985 US 774118**

(43) Date of publication of application:
**04.03.1987 Bulletin 1987/10**

(73) Proprietor: **ENZO BIOCHEM, INC.**
**New York, N.Y. 10013 (US)**

(72) Inventor: **Rabbani, Elazar**
**New York, N.Y. 10003 (US)**

(74) Representative: **VOSSIUS & PARTNER**
**Postfach 86 07 67**
**81634 München (DE)**

(56) References cited:
**EP-A- 0 032 286        EP-A- 0 075 379**
**EP-A- 0 078 965        EP-A- 0 178 790**
**EP-A- 2 304 939        EP-A- 3 966 897**
**US-A- 3 992 158        US-A- 4 274 832**
**US-A- 4 381 921        US-A- 4 391 904**

EP 0 212 670 B2

**Description**

This invention relates to a method for localizing a signal to enhance the detection of an analyte moiety. More particularly, it relates to a method for localizing a signal by generating the signal in a medium that comprises a material that limits the spread of the signal from the point of origin of the signal.

Assays for determining the presence of analyte moieties by means of analyte-specific moieties are well known in the art. The methods generally comprise fixing the analyte moieties in a sample to a support, blocking areas of the support to which no analyte moieties are fixed with a blocking agent, and contacting the sample with analyte-specific moieties to form analyte moiety/analyte-specific moiety complexes. The analyte-specific moieties generally have signalling moieties attached to them. The non-bound analyte-specific moieties are separated from ones that are bound, by washing, for example, and the support is contacted with a solution containing a precursor that can be converted by the signalling moieties into a signal. The generation of the signal (usually a chromogenic or fluorescent product) indicates the presence of the analyte moieties. Generation of the signal can be by means of a precipitable or soluble assay.

A precipitable assay concentrates the signal at the site of generation, a result which makes this assay particularly advantageous for in situ detection by permitting one to visualize the signal in relation to the cell structure. Such an assay, however, has limitations in that precipitates may not form if the amount of signal formed does not exceed the maximum solubility of the signal in the solution volume (giving a false negative result), and in that precipitates are not readily quantifiable. Furthermore, it is believed that the formation of the precipitate at the site of signal generation significantly reduces enzyme turnover rate (because the precipitate blocks the enzyme active site), thus decreasing the effective half-life of the enzyme.

A soluble assay produces a signal that can be quantified by, for example, spectrophotometric or colorimetric methods. This assay has a limitation in that the signal may escape detection if the signal is generated at a slow rate and spreads (distributes over a given volume) before it can be detected.

Spread of molecules from one area of a solution to throughout the solution is mainly due to convection and diffusion. Generally, most of the spread is attributable to convection which is defined as the circulatory motion that occurs in a fluid at a nonuniform temperature owing to the variation of its density and the action of gravity. Convection enhances spread of molecules by causing mass movement of the areas of the solution containing the molecules. Since convection involves bulk movement, molecules will spread more rapidly in a solution because of convection than because of diffusion. Thus, the decreased sensitivity of detection in both precipitable and soluble assays is due mainly to spread of the signal by convection.

Some spread of molecules is attributable to diffusion. Diffusion is defined as the net flow of a region of high concentration to one of low concentration when there is no driving force. Diffusion is thus the migration of molecules through random movement.

One parameter that greatly affects diffusion in solution is the solvent viscosity. Viscosity is defined as the effect generated by friction as a result of the impediment of the movement of a liquid across a surface.

The addition of particular solute molecules to a solvent with viscosity $n_o$, yields a solution of higher viscosity, n. This can be thought to result from increased friction between adjacent unimolecular liquid planes caused by the fact that the particular solute molecules are larger than the solvent molecules and hence extend through several of these hypothetical planes. The ability of a solute molecule to alter solvent viscosity depends on several parameters, namely, the volume of the occupied solution, the ratio of length to width of the molecule, and the rigidity of the molecule. Viscosity is frequently expressed as the specific viscosity, $n_{sp}$, which is defined as the fractional change in viscosity produced by the addition of the solute molecules as shown:

$$n_{sp} = \frac{n - n_o}{n_o}$$

The present invention relates to a method for localizing a signal. The signal is generated in an assay carried out to detect the presence of an analyte moiety.

The signal remains substantially localized because it is generated in a medium that comprises a material that limits the spread of the signal from the point of origin of the signal. The media that are preferred for practicing the method of the present invention are referred to herein as specific, indicative, viscous, or thick media. The specific medium comprises a solution, a network, and a signal precursor. The indicative medium comprises a solution, a network, and a signalling moiety. The viscous medium comprises a solution, a component that imparts the desired viscosity to the medium (hereafter referred to as the specific component), and a signal precursor. The thick medium comprises a solution, a specific component, and a signalling moiety. The network and specific component limit the spread of the signal from the point of origin of the signal. The network is suspended or dissolved in the solution, while the specific component is dissolved in the solution. The signal is generally a fluorescent or chromogenic compound.

One embodiment of the present invention relates to: A method for detecting the presence of an analyte moiety comprising the steps of:

(a) fixing to a support either a first analyte moiety or a first analyte-specific moiety;
(b) forming a complex comprising either:

(1) said first analyte moiety and a second analyte-specific moiety, or
(2) said first analyte-specific moiety and a second analyte moiety, wherein each of said second analyte-specific moiety and said first analyte-specific moiety has a signalling moiety capable of generating a signal either directly or indirectly attached thereto;

(c) forming either a specific or viscous medium on said support, wherein said specific medium comprises (1) a solution, (2) a polymer network, and (3) a signal precursor, and wherein said viscous medium comprises (1) a solution, (2) a specific component that imparts the desired viscosity to said viscous medium, and (3) a signal precursor; and
(d) generating a signal by means of said signalling moiety.

Another embodiment of the present invention relates to: A method for detecting the presence of an analyte moiety in a sample, wherein said analyte moiety is or comprises either a signalling moiety or a signal precursor, comprising the steps of:

(a) forming either a:

(1) specific or viscous medium on a support when said analyte moiety is or comprises a signalling moiety capable of generating a signal directly or indirectly, wherein said specific medium comprises (a) a solution, (b) a polymer network, and (c) a signal precursor, and wherein said viscous medium comprises (a) a solution, (b) a specific component, and (c) a signal precursor, or
(2) indicative or thick medium on a support when said analyte moiety is or comprises a signal precursor, wherein said indicative medium comprises (a) a solution, (b) a polymer network, and (c) a signalling moiety, and wherein said thick medium comprises (a) a solution, (b) a specific component, and (c) a signalling moiety;

(b) contacting one of said media with said analyte moiety; and
(c) generating a signal by means of said signalling moiety.

## I. THE USE OF A SPECIFIC OR INDICATIVE MEDIUM

One embodiment of the present invention relates to a method for detecting an analyte moiety by means of a signal generated in a specific medium. The specific medium comprises at least three components: a network, a solution, and a signal precursor. The term network refers to a three-dimensional shaped structure pervading the medium. The term solution refers to the liquid present in the medium. The term signal precursor refers to the compound that is converted into a signal. A variation of this embodiment relates to a method for detecting an analyte moiety in an indicative medium. The indicative medium comprises a network, a solution, and a signalling moiety.

The network is formed as a result of the presence of polymers in the solution. The network can be continuous or it can be discontinuous. A continuous network is present when the network is comprised of one integral polymer. A discontinous network unit is present when the network is comprised of more than one integral polymer. The monomers in each integral polymer are linked covalently.

Polymers that provide a network can be comprised of organic monomers, inorganic monomers, or a combination of organic and inorganic monomers. The polymer can be linear or branched. Large polymers can be ground to any shape, although the bead shape is preferred. The bead can be spherical, and can be porous or non-porous. Particularly preferred is the spherical porous bead. The polymer can be suspended or dissolved in the solution.

Networks comprising polymers containing organic monomers that generally are suspended in the solution include, for example, carbohydrates, polysaccharides, starches, liposaccharides, dextrans, methylcelluloses, mucilages, plant gums, celluloses, agaroses, Sepharoses® , polyacrylamides, and liquid crystals. Preferred are the agarose, Sepharose®, and dextran polymers. Polymers containing inorganic monomers that are generally suspended in the solution include, for example, zeolites, silicates, phosphates, sulfates, and aluminates. Preferred are the silica and alumina polymers.

Networks comprising polymers containing organic monomers that generally are dissolved in the solution include, for example, carboxymethyl cellulose, polyvinyl alcohol, polyglycols, glycols, proteins, polypeptides, pectins, mucins, lipoproteins, DNA, RNA, and polynucleotides. Preferred polymers are the carboxymethylcelluloses, polyglycols, proteins, polypeptides, DNA, RNA, polynucleotides, mucins and pectins. More preferred polymers are the carboxymethylcelluloses, proteins, DNA, RNA, polynucleotides, mucins and pectins.

Polymers that contain organic and inorganic monomers that are generally dissolved in the solution include, for example, polymers comprising bifunctional organic monomers cross-linked by bivalent metals. The bifunctional groups include, for example, aldehydes, ketones, alcohols, carboxylic acids, amines, amides, and thiols.

While I do not wish to be bound by theory, I believe that the network reduces bulk movement of solution portions of the medium, thus reducing the convection in the medium. The reduction in convection decreases the rate of spread of the signal, thereby resulting in substantial localization of the signal. Localization of the signal provides the assay with increased sensitivity.

When the network comprises a polymer suspended as a solid in the solution, the solid network provides the barrier which reduces the bulk movement of the solution. When the network comprises a polymer dissolved in the solution, the network that provides the barrier which reduces the bulk movement of the solution arises from the non-covalent association, interaction, linking, and binding of the dissolved polymers to each other. The non-covalent interaction can be by, for example, hydrogen bonding, ion pairing, or ionic attraction.

It is believed that the network can further localize the signal by interacting with the signal. The interaction can be non-covalent or covalent. Non-covalent interaction is generally by electrostatic interaction, which includes, for example, hydrogen bonding, ion pairing, dipole-dipole interaction, and Van der Waals attraction. Covalent interaction is by the formation of a bond between the network and the signal.

Networks that can interact non-covalently with the signal comprise the various polymers containing functional groups or polymers coated with a compound containing functional groups. They include, for example, those used for normal phase chromatography such as alumina, silica gel, and carbohydrates, those used for reverse phase chromatography, such as octadecyl- and phenyl-bonded silica, and those used for gas chromatography, for example, diatomaceous earth coated with silicone oil or polyethelene glycol esters. An example of non-covalent interaction between the network and the signal is the network comprised of octadecyl-bonded silica and the signal is a phenylene diamine. The organic octadecyl groups bind the organic o-phenylene diamine molecule non-covalently, providing additional signal localization.

Networks that can interact covalently with the signal comprise, for example, those that contain a functionality that can react with a signal, for example, as by the formation of a Schiff base. An example of covalent interaction between the network and the signal is where the precursor used in an assay is an aromatic dye comprising an alpha diphosphorylated gem diol, the network in the medium is a polymer comprising a primary amino group, and the signalling moiety attached to the analyte-specific moiety is alkaline phosphatase. The cleavage of the phosphate groups from the gem doil, by the enzyme eliminates a molecule of water and forms an aldehyde-containing fluorescent signal. The aldehyde group on the signal can react with the amino group on the network to form a Schiff base, thus immobilizing the signal to the network, and providing additional signal localization.

This invention also contemplates signal generating assays in a medium on a support wherein the support interacts with the signal and provides additional signal localization. The interaction can be covalent or non-covalent as described hereinabove wherein tie network interacts with the signal.

The solution component of the medium can be any liquid compatible with the particular assay and the network. Aqueous solutions or organic solutions can be used for signalling moieties which are catalysts, for example, enzymes. Preferred are the aqueous solutions. See Enzymatic Catalysis In Organic Media at 100° C, A. Zaks and A.M. Klibanov, Science. 224, 1249-1251 (1984), and Preparative Production of Optically Active Esters and Alcohols using Esterase-Catalyzed Stereospecific Transesterification In organic Media by B. Cambuv and A. M. Klibanov, J. Amer. Chem. Soc 106, 2687-2692 (1984).

The method of this invention is adaptable to both precipitable and soluble assays. For precipitable assays, the reduction in spread by the network allows the signal to concentrate in a small volume, i.e. signal localization, and thus exceed the signal solubility in the solution so that a visible precipitate is formed. For soluble assays, the reduction in spread by the network allows the signal molecules to remain concentrated so that the signal, for example, fluorescence, luminescence, or chromogen can be more readily observed.

The specific medium of the present invention is prepared by suspending or dissolving in an aqueous or organic solution the polymer that forms the network. At times, it may be necessary to heat the medium and then cool it to ensure uniform network distribution. The specific medium can optionally further comprise a buffer.

When the network is suspended in the medium, a two phase system results. When the network is dissolved in the medium, a one phase system results. Generally, a two phase system comprises a solid and liquid phase. The liquid phase is dispersed in and between the network. A two phase system can, however, comprise two liquid phases as when a first liquid phase is emulsified in a second liquid phase. The units forming the network are then dispersed by means of the first liquid phase.

The concentration of the network in the solution required by this invention depends on the particular network and the particular solution. For polymeric networks which are suspended in the solution, the amount of solution present will be dictated, for example, by the size, shape, and swelling of the polymer. The swelling of the polymer is generally controlled by the percent of cross-linkage, with the swelling being inversely proportional to the percent of cross-linkage. Highly cross-linked polymers contain high ratios of network to solution, as compared to low cross-linked polymers and thus form tight beads. Tighter beads can be advantageous in reducing spread of a signal because they can provide a dense network layer. Low cross-linked polymers contain high ratios of solution to network as compared to highly cross-linked polymers and form soft beads which are advantageous when large amounts of solution are desired, for example, when the precursor is relatively insoluble in the solution. Preferred suspended networks are gels. Tables for determining the correlation between cross-linkage and swelling of the polymer are readily available. See the pamphlets Ion-Exchange Chromatography, and Gel Filtration Chromatography, by Pharmacia Chemical Company, Piscataway, New Jersey.

For polymeric networks which are dissolved in the solution, the polymer should generally comprise from about one percent to about fifty percent, preferably, from about two percent to about thirty percent, and more preferably, from about five percent to twenty percent of the solution (weight/volume). High concentrations of polymer provide a very elaborate network and can significantly reduce the spread of signal as compared to low concentrations of polymers. However, high concentrations of polymers can make the medium very viscous, thus making it difficult to pour or layer the medium over a support. Thus, optimum concentration of the polymers in the solution will thus depend on the type of assay to be carried out. This is best determined by preliminary control experiments which can be readily carried out by those skilled in the art.

An example of a medium where the network is formed by a dissolved polymer is where 2% carboxymethylcellulose (CMC) (w/v) is heated in water to about 50°C to form a clear medium, and the medium is then permitted to cool. The medium remains clear, and the soluble network is uniformly dispersed in the medium. An example of a medium where the network is formed by a suspended polymer is where 2% agarose is heated in water to about 60°C to form a clear medium, and the medium is then permitted to cool. Upon cooling, the swollen agarose separates and settles out of the water as beads. The swollen beads and the water entrapped in and between the beads form a medium comprising an insoluble network.

## II. THE USE OF A VISCOUS OR THICK MEDIUM

A second embodiment of the present invention relates to a method for detecting an analyte moiety by means of a signal generated in a viscous medium. The viscous medium comprises at least three components: a specific component, a solution, and a signal precursor. The term specific component refers to the material that imparts a viscosity of at least 1.5 mPa·s (centipoise (cp)) to the medium. The term solution refers to the solvent and other liquids in the medium. The term signal precursor refers to the substance that is converted by means of the signalling moiety into a signal. A variation of this embodiment relates to a method for detecting an analyte moiety in a thick medium. The thick medium comprises a specific component, a solution, and a signalling moiety.

While I do not wish to be bound by theory, I believe that solutions of high viscosity create a barrier against the diffusion of molecules. Generally, the higher the viscosity of the solution, the greater will be the diffusion barrier. Determination of the presence of an analyte moiety often depends on determining the presence of a signal. When the signal is a chromogenic or fluorescent compound, reducing the diffusion of this signal by increasing the viscosity of the solution will result in localization of the signal in one area and will enhance its detection by visual or instrumental methods.

The specific component can be any molecule that is soluble in the solution and able to increase the viscosity of the medium. A viscous or thick medium, for the purpose of this invention, is defined as one having a viscosity of at least 1.5 mPa·s (cp). The viscosity of the medium can, however, range from about 1.5 mPa·s (cp) to about 1,000 mPa·s (cp). The diffusion of a signal is not significantly retarded in a medium whose viscosity is lower than about 1.5 cp. A medium whose viscosity is greater than about 1000 mPa·s (cp) may hinder the diffusion of the precursor and may not be capable of being layered or poured over the support. However, media with a viscosity higher than 1000 cp can be used if the medium can be layered over the support and the precursor can diffuse to the signalling moiety. As is shown by the equation, supra viscosity is dependent on the temperature. It is contemplated that the viscosity of the medium as claimed by this invention is ascertained at the time when the signal is generated in the medium. Thus, whether the assay is carried out at room temperature, 37°C, at 0°C, or any other temperature, it is at this temperature at which the viscosity of the medium should be at least about 1.5 mPa·s (cp) and preferably from about 1.5 mPa·s (cp) to about 1000 mPa·s (cp).

The specific component should not materially affect the binding of the analyte-specific moiety either to the support or to the analyte moiety, and should also not affect the reaction of the signalling moiety with the precursor.

The concentration of the specific component required to produce a viscosity of about 1.5 mPa·s (cp) or higher will vary with the particular specific component. There are tables available which disclose the viscosities of solutions containing varying concentrations of different specific components. See for example Bingham and Jackson, Bureau Standards Bulletin, 14, 59 (1918), ML. Sheeley, Ind. Eng. Chem., 24, 1060 (1932), and Segur and Oberstar, Ind. Eng. Chem., 43, 2117 (1951). For other specific components that are useful in increasing the viscosity of solution, the amount of required material can be readily determined by one skilled in the art by gradually adding the material until the desired viscosity, as measured, for example, by a viscometer, is obtained.

Specific components suitable for this invention comprise monomers, oligomers, and polymers soluble in the solution. The specific components include, for example, sugars, alcohols, glycols, proteins, polypeptides, amino acids, deoxynucleic acids, ribonucleic acids, polynucleotides, nucleotides, nucleosides, bases, fatty acids, pectins, mucins, polysaccharides, steroids, flavanoids, alkaloids, terpenes, vitamins, co-enzymes, and prostaglandins, hydrocarbons, aromatic hydrocarbons, and porphyrins.

Preferred specific components include, for example, sugars, glycols, proteins, polypeptides, amino acids, deoxynucleic acids, ribonucleic acids, polynucleotides, nucleotides, nucleosides, bases, pectins, mucins, and polysaccharides. Particularly preferred are sugars, glycols, proteins, deoxynucleic acids, ribonucleic acids, polynucleotides, pectins, mucins, and polysaccharides.

Some specific components in addition to retarding diffusion of the signal by increasing the viscosity of the medium, can also decrease spread through convection by forming a network in the medium. An example of such a specific component is carboxymethylcellulose.

The specific component retards the diffusion of the signal by increasing the shear gradient or shear rate of the medium which in effect serves to increase the viscosity of the medium. The extent of increase in the viscosity of the medium by the specific component depends on the axial ratio, shape, and size of the specific component. The viscosity of a medium comprising a specific component can be determined by methods known in the art. Such methods include, for example, an Ostwald capillary viscometer, a Ubbelohde capillary viscometer, a Couette viscometer, a Zimm-Crothers Floating-rotor viscometer, and a Cartesian-diver rotating-cylinder viscometer. See for example Physical Biochemistry by David Freifelder, pp. 359-363.

The viscous or thick medium of the present invention may be prepared by suspending or dissolving in an aqueous or organic solution, an amount of the specific component that produces a medium comprising a viscosity at least about 1.50 mPa·s (cp). For some rather insoluble specific components, it may be necessary to initially heat the solution and then permit it to cool in order to ensure uniform specific component distribution. The viscous or thick medium can optionally further comprise a buffer.

Media of high viscosity will be more effective in localizing a signal than media of low viscosity; but high viscosity media may retard the diffusion of the signal precursor, and thus prevent the signalling moiety from rapidly contacting and reacting with the signal precursor. Thus, the actual viscosity chosen for the medium depends on the particular assay. The optimum viscosity for a particular assay can readily be determined by a person skilled in the art using standard methods.

### III. THE OTHER COMPONENTS OF THE MEDIA

The solution part of the medium can be any liquid compatible with the analyte moiety, analyte-specific moiety, signalling moiety, specific component, and the precursor. Aqueous solutions or organic solutions can be used for signalling moieties which are catalysts, for example, enzymes. Preferred are the aqueous solutions. See Enzymatic Catalysis In Organic Media at 100°C, A. Zaks and A.M. Klibanov, Science. 224, 1249-1251 (1984), and Preparative Production of Optically Active Esters and Alcohols using Esterase-Catalyzed Stereospecific Transesterification In Organic Media by B. Cambuv and A.M. Klibanov, J. Amer. Chem. Soc. 106, 2687-2692 (1984).

The signal precursor is the compound that is converted into the signal. The signal precursor is usually converted to a signal by an oxidation or hydrolysis reaction that increases the resonance structure of the precursor. Oxidation generally involves dehydrogenation of the signal precursor. Hydrolysis generally involves removing a fragment from the precursor to generate an aromatic hydroxyl, aromatic amine, thiol, or carbonyl group. The precursor can be added prior to or following the addition of the component. If the precursor is stable to heat and not readily soluble in the particular medium, the precursor can be often solubilized by heat.

Signal precursors comprise, for example, the various dyes. An example of a signal precursor that is converted into a signal by oxidation is diaminobenzidine, and an example of a signal precursor that is converted into a signal by hydrolysis is bromochloroindolyl phosphate (BCIP).

The signalling moiety generates the signal. The signalling moiety can be, for example, a catalyst, e.g. an enzyme, a radical generator, or an oxidizing agent. Preferred signalling moieties are the catalysts and enzymes because one catalyst or enzyme can ganerate many signals from precursors. Specific examples of signalling moieties are the phosphatase, esterase, dehydrogenase, and peroxidase enzymes, porphyrin coenzymes, catalytic cyclodextrins, alkyl peroxides, and alkyl persulfates.

The signalling moiety is attached to the analyte-specific moiety. The signalling moiety can be attached to the analyte-specific moiety prior to contacting the analyte-specific moiety to the analyte moiety or following contacting the analyte-specific moiety to the analyte moiety. The signalling moiety can be attached to the analyte-specific moiety covalently or non-covalently. Covalent binding can be, for example, via an isothiocyanate functionality, while non-covalent binding can be, for example, via hydrogen bonding. See Ward et al., EP-A 63,879, published on November 3, 1982, based on the priority document that issued as U.S. patent No. 4,711,955. See also Engelhardt et al., EP-A 97,373, published on January 4, 1984.

The signal must have a detectable characteristic not present in the precursor. The chracteristic can be fluorescence emission, luminescence emission, ultraviolet absorption, or visible color.

The signal is generated by the signalling moiety directly or indirectly. Direct generation means that the signalling moiety converts the signal precursor to a signal. Indirect signal generation means that the the signalling moiety converts a first substance to a second substance, and the second substance either converts the signal precursor to a signal, or else converts a third substance to a fourth substance, in wnich latter case, the fourth substance converts the signal precursor into the signal.

An example where a signalling moiety acts directly to convert a signal precursor to a signal (direct conversion) is where alkaline phosphatase (signalling moiety) converts p-nitrophenyl phosphate (signal precursor) to p-nitrophenol (signal). An example of where a signalling moiety acts indirectly is where a signalling moiety decomposes a substance to form radicals and the radicals act to convert the signal precursors to signals. Preferred substances for use in such indirect conversions include peroxides and persulfates. Hydrogen peroxide is particularly preferred. An example of this indirect conversion is where horseradish peroxidase converts hydrogen peroxide to hydroxyl ions, and the hydroxyl ions convert NADH to NAD$^+$. An example of a still more indirect conversion is where a hydroxyl ion is not used, as above, to convert NADH to NAD$^+$, but rather converts potassium iodide to iodine and the iodine converts polyethylene glycol to a polyethylene glycol-iodine complex.

It can be advantageous in some instances to have the signalling moiety generate a signal indirectly instead of directly. This can be when the medium is very thick and viscous, and the signal precursor is a rather large molecule. A large molecule will diffuse more slowly in such a medium than a small molecule. Since direct signal generation depends on contact of the signal precursor with the signalling moiety, if the signal precursor is a large molecule, the retardation in diffusion of this signal precursor molecule can effectively decrease the rate of signal generation. In these instances the use of a small molecule as a first substance which can more readily diffuse to the signalling moiety and become converted into a second substance which in turn can then readily diffuse to the signal precursor and convert the latter into a signal, may provide for greater signal generation.

The method of this invention is adaptable to both precipitable and soluble assays. For precipitable assays, the retardation in diffusion allows the signal to concentrate in a small volume and thus exceed their solubility in the solution so that a visible precipitate is formed. For soluble assays, the retardation in diffusion allows the signal molecules to remain concentrated so that fluorescence, luminescence, or chromogen can be more readily observed.

## IV. DESCRIPTION OF OTHER MOIETIES

The analyte moiety is the substance that is to be detected in the assay. Detection is by means of an analyte-specific moiety that forms a complex with the analyte moiety. The analyte moiety must have an informational portion that an analyte-specific moiety can recognize by means of a complementary structure. The informational portion refers to the sequence and/or arrangement in space of the analyte moiety that makes the analyte moiety distinguishable and recognizable. Analyte moieties include, but are not limited to, microorganisms, fungi, algae, plant cells, animal cells, tumor cells, ligands, polysaccharides, polypeptides, nucleic acids, and polynucleotides. The analyte moiety can be derived from serum, tissue extracts, cell smears, urine, sputum, feces, saliva, puss, semen, fermentation broth, culture media, water aliquots, environmental samples, and foods.

The analyte-specific moiety recognizes and binds to a specific analyte moiety. The analyte-specific moiety can be a, for example, ligand, receptor, glycoprotein, protein, enzyme, enzyme substrate, enzyme inhibitor, antigen, hormone, antibody, polynucleotide, amino acid, lipid, and lectin. Preferably, it is a polynucleotide or oligonucleotide.

## V. METHODS FOR CARRYING OUT THE INVENTION

An assay in which this invention can be used typically comprises, for example, fixing a sample containing the analyte moieties, to a support, for example, to a nitrocellulose membrane. The support is then contacted with a solution containing a blocking compound, for example bovine serum albumin (BSA). The blocking compound binds to sites on the support other than where the sample is bound to. This ensures that the analyte-specific moieties (defined below) become attached to the support only if the sample contains the analyte moieties. Further details of such assays can be found in the articles by Wie-Shing Lee and James L. Bennington, Recombinant DNA Technology: Some Applications in Clinical Microbiology, Laboratory Management, pp 21-26, (April 1985), C.J. Stanley, F. Paris, A. Plumb, A. Webb, and A. Johannsson, Enzyme Amplification: A New Technique For Enhancing the Speed and Sensitivity of Enzyme Immunoassays, American Biotechnology Journal, pp. 48-53 (May/June 1985), and Robert H. Yolken, Enzyme Immunoassays for the Detection of Infectious Antigens in Body Fluids: Current Limitations and Future Prospects, Review of Infectious Diseases, 4, pp. 35-68; D.E. Kennell, Principles and Practices of Nucleic Acid Hybridization, Proc. Natl. Acad. Res. Mol. Biol. 11: 259-301 (1971); R.H. Volken, Enzyme Immunoassays for the Detection of Infectious Antigens in Body Fluids: Current Limitations and Future Prospects, Reviews of Infectious Diseases, 4, 35-68 (1982), and Enzo Biochem, Inc., Catalog 1985.

The support is then contacted with a solution containing the analyte-specific moieties to permit the analyte-specific moieties to attach to any analyte moieties present. The analyte-specific moiety specifically recognizes and binds the particular analyte moiety. A signalling moiety is attached to the analyte-specific moiety. Methods for attaching a signalling moiety to an analyte-specific moiety can be found in European Patent Publication 0,638,79 by Ward, et al., published November 3, 1982, based on the priority document that issued as U.S. Patent No. 4,711,955, supra. See also European Patent Publication 0,097,373 by Engelhardt, et al., published January 4, 1984, and in the article by Yolken referenced hereinabove. A washing solution which can be water or a buffer at about pH 7.0 is used to remove all unbound analyte specific moieties from the support.

An assay can also comprise, for example, immobilizing the analyte-specific moieties on a support and contacting the sample containing the analyte moieties with the support. The support is then blocked with a blocking agent and second different analyte-specific moieties that are specific for the same analyte moieties are contacted with the support (generally known as the sandwich assay). A signalling moiety is attached to the second analyte-specific moiety.

The support containing the analyte-specific and signalling moieties is placed in a container. The container can be, for example, a tray, a cup, or a dish. The medium is layered over the support. The medium can be layered over the support as a film or a slab as when the network is formed by polymeric units insoluble in the solution, or the medium can be layered over the support as a solution as when the network is formed by monomeric, oligomeric, or polymeric units soluble in the solution.

The layering of the medium over the support brings a substance upon which the signalling moiety can act into contact with the signalling moiety. This leads to the generation of a signal, as explained hereinbelow, which generally is fluorescent or chrornogenic. While I do not wish to be bound by theory, I believe that the spread of the signal by convection in a specific or indicative medium is reduced because of the network structure in the medium and the spread of the signal by diffusion in a viscous or thick medium is reduced because of the viscosity of the medium, resulting in a signal that therefore remains localized for relatively long periods of time. The actual duration of signal localization depends on the medium used. The tighter and the stronger the network, the greater the reduction in the spread, and the greater the duration of signal localization.

When a viscous medium is used, diffusion of the signal is limited because of the presence of the specific component, resulting in signal localization. The actual duration of signal localization depends on the specific component used. The greater the viscosity, the greater the retardation in diffusion, and the greater the duration of signal localization.

This method of signal localization can be conveniently carried out, for example, with Enzyme Multiplied Immunoassay Techniques (EMIT), Enzyme-linked Immunosorbent Assays (ELISA), and in situ hybridization methods. The analyte-specific moiety containing the signalling moiety can be used in competitive binding assays, sequential saturation heterogeneous formats, and "sandwich" heterogeneous formats. These methods are well known to one skilled in the art. See U.S. Patent document 4,238,195 of R.C. Boguslaski and R.J. Carrico.

The method of signal localization can also be used to detect an analyte wherein the analyte moiety is or comprises a signalling moiety. This can be, for example, when the analyte is or comprises an enzyme, oxidizing agent, or reducing agent. In these instances, an analyte-specific moiety is not required. A signal precursor for the signalling moiety is present in the medium. Upon contact and reaction of the signalling moiety, an enzyme, for example, with the signal precursor, a color or fluorescence (signal) is generated that remains localized in the medium. The signalling moiety can act directly on the signal precursor or indirectly as described hereinabove.

Similarly, the method can be used to detect an analyte moiety which comprises a signal precursor that can be converted to signal by means of a signalling moiety present in the medium. The signalling moiety can be, for example, an enzyme, oxidizing agent, or reducing agent, while the signal precursor can be, correspondingly, a substrate, an oxidizible substance, or a reducible substance. A signal is generated upon the reaction of the signalling moiety with the signal precursor. This method is suitable where the analyte moiety is or comprises an enzyme, for example, a peroxidase, a deaminase, an oxidoreductase, an esterase, a phosphatase, or an isomerase. Preferred analyte moieties are those that are or comprise peroxidases. These enzymes decompose peroxides to generate oxygen which can oxidize a signal precursor to a signal such as chromogen.

Examples of peroxides suitable for use with peroxidase enzymes are those of polyhydroxyl compounds such as, for example, mannitol peroxide and sorbitol peroxide. Examples of signal precursors that can be converted into signal in the presence of peroxidases and peroxides are dyes such as guaiac and 2,2'-azino-bis (3-ethylbenzthiazoline sulfonic acid) (ABTS).

The peroxidase in the analyte moiety can be from any cellular component comprising a peroxidase-mediated system. An example of an application where the analyte moiety comprises a signalling moiety is for the detection of blood in feces. Currently a widely used method employs a test strip impregnated with guaiac as the chromogen in a peroxidase-like reaction of blood. The test, in common kit form, comprises filter paper impregnated with guaiac and a separate container of hydrogen peroxide. This necessitates separate packaging of the peroxide. The quality and stability of the peroxide in its separate container must be guaranteed by the supplier. Because the peroxide-containing container is separate from the test strip it is often misplaced and therefore is unavailable when needed to perform the test. To carry out the test, a feces sample is applied to the guaiac test paper and the solution of hydrogen peroxide is then applied to the reverse side of the paper. The formation of a blue ring indicates the presence of blood. This disclosure describes a formulation which would incorporate both the guaiac or other chromogen and a stabilized hydrogen peroxide into a medium. A specific or viscous medium can be used, preferably, a specific medium. It is preferred that the network in the specific medium be generated by means-of a gel. This eliminates the need for separate packaging of reagents, and separate addition of the hydrogen peroxide, thereby simplifying the test procedure.

Detection can be carried out with the use of an indicative or thick medium where the analyte moiety is or comprises a signal precursor. In this instance, a signalling moiety is added to the medium. The network or specific component of the medium causes the signal to remain localized.

The methods of the present invention will be more clearly understood from the following examples which are by way of demonstration and not by way of limitation. Examples I-V exemplify the first embodiment of this invention while example VI-VIII exemplify the second embodiment of this invention.

### EXAMPLE I:

Sweet potato acid phosphatase was diluted serially 1:10 in phosphate buffered saline (PBS) containing 0.1% (w/v) bovine serum albumin (BSA). (The BSA had been previously treated with acid to destroy any contaminating phosphatase). The dilutions ranged from 1 unit/ml to $1 \times 10^{-7}$ units ml. One microliter aliquots of each enzyme dilution were spotted on nitrocellulose membrane strips that had been rinsed with distilled water, and dried.

The specific medium was prepared by adding agarose to give a concentration of 1.5% to an 0.2M sodium acetate solution, pH 5.5, and heating the solution to $80C^\circ$ dissolve the agarose. The resulting medium was cooled to approximately $40^\circ C$, and the precursor 7-methyl umbelliferyl phosphate was then added (1 mg/ml). The clear medium was layered over the enzyme-dotted strips and allowed to cool to form an agarose gel.

This agarose gel was periodically observed with a long wavelength-emitting mineral lamp. A fluorescent signal was observed in the agarose gel above the spot containing $10^{-5}$ units of enzyme within about 15 minutes.

A control nitrocellulose strip dotted with the same enzyme solutions, but incubated in a solution containing 1 mg/ml of 7-methyl umbelliferyl phosphate in 0.2M sodium acetate, pH 5.5 without agarose showed no localized fluorescence around the dots. Instead, a diffuse fluorescent solution appeared.

### EXAMPLE II:

Alkaline phosphatase ranging in amounts from $10^{-3}$ units to $10^{-9}$ units was dotted down in sequence on each of eight nitrocellulose membrane strips. Flavone diphosphate, a precursor, was dissolved in the following solutions to give a final concentration of 0.5 mg/ml:

A. 0.1M Tris, pH 8.8, in 2M NaCl
B. 0.1M Tris, pH 8.8, 0.75% agarose, in 2M NaCl
C. 0.1M Tris, pH 8.8, 0.4% agarose, 1.0% carboxymethylcellulose (CMC), in 2M NaCl.
D. 0.1M Tris, pH 8.0, 2% CMC
E. 0.1M Tris, pH 8.5, 2% CMC in 2M NaCl
F. 0.1M Tris, pH 8.8, 1.5% CMC in 2M NaCl
G. 0.1M Tris, pH 8.8, 1% CMC in 2M NaCl
H. 0.1M Tris, pH 8.8, 0.5% CMC in 2M NaCl

The agarose in the examples formed an insoluble network, while the CMC formed a soluble network. The specific medium comprised the precursor, the solution, and either the agarose or the CMC. The nitrocellulose strips were placed in one of the above media and the signal allowed to develop at room temperature for various periods of time as described below. The results can be summarized as follows:

1. Fluorescence was observed in all strips immediately following contact with the substrate.
2. The signals in media B and C were more intact then the others. They appeared to be of the same intensity from about hours to 18 hours; after contact, but the signal in B became more diffuse after 18 hours.

3. The signals in D to H were diffuse, as in A; no difference was observed between D and E.

4. Carboxymethylcellulose (CMC) in concentrations greater than 0.5% formed a viscous, transparent, homogenous solution upon swelling. Solutions containing less than 0.5% of CMC separated into two phases, with the CMC settling on the bottom.

## EXAMPLE III:

Calf intestinal alkaline phosphatase was diluted serially 1:10 in water and applied to small squares of nitrocellulose paper in 1 ul aliquots. The amount of applied enzyme ranged from $10^{-1}$ units to $10^{-4}$ units in each series. The enzyme-containing squares were placed in individual wells of a multiwell polystyrene plate. Each enzyme dilution series was carefully overlayed with a mixture comprising 1 mg/ml of 4-methyl-7-hydroxycoumarin phosphate, (sodium salt), dissolved in one of the following solutions:

A. 0.1M Tris HCl pH 8.8
B. 0.1M Tris HCl pH 8.8 in 50% (v/v) glycerol: $H_2O$
C. 0.1M Tris HCl pH 8.8 in 50% (w/v) sucrose: $H_2O$
D. 1.5% (w/v) agarose in A
E. 1.5% (w/v) agarose in B
F. 1.5% (w/v) agarose in C

The media were allowed to incubate in the plate undisturbed at room temperature. The phosphatase generated product was visualized as blue fluorescence using a long wavelength mineral lamp at various times. Localization of the soluble product 4-methyl-7-hydroxycoumarin was noted at the highest enzyme concentration. The results are summarized in tables I and II:

TABLE I

| Localization of Signal in Various Substrate Media | |
|---|---|
| Substrate Media | Localization of Signal with $10^{-1}$ Units Enzyme |
| A | Localized "dot" on nitrocellulose appear after 15 seconds and immediately began to disperse in a non-random fashion. After 1 hour, the fluorescence was uniformly distributed throughout the medium and no localized signal was detectable. |
| B + C | The localized dot appeared after 15 seconds. The dispersal of fluorescence into the medium occurred initially by convection and was retarded with respect to A. After 1 hour, some localized signal on the nitrocellulose was seen through the generally dispersed fluorescence of the medium. |
| D | A localized dot appeared after 15 seconds. Dispersal of fluorescence was evident after 10 minutes and occurred in a random manner forming an expanding dome-shaped fluorescent region. Dispersion appeared to be by diffusion. |
| E + F | Localized dot appeared after 15 seconds. After 1 hour, E remained localized on the nitrocellulose with little evidence of diffusion. F showed some dispersion into the media above the dot as a result of diffusion. |

TABLE II

| Localization of Signal Generated by Varying Enzyme Concentrations After 1 Hour. | | | |
|---|---|---|---|
| Media | Units of Enzyme | | |
| | $10^{-2}$ | $10^{-3}$ | $10^{-4}$ |
| A | - | - | - |
| B | ± | - | - |
| C | ± | - | - |
| D | + | - | |
| E | ++ | ± | - |
| F | ++ | + | - |

## EXAMPLE IV:

2.0 ml of the following solutions are introduced into 10mm X 75 mm glass culture tubes:

1. Water
2. 1% (w/v) agarose in water
3. 1% (w/v) agarose in 50% (w/v) glycerol:water
4. 1% (w/v) agarose in 50% (w/v) sucrose:water

The agarose containing gels (2-4) are allowed to harden at room temperature. Fifteen µl of a solution of rhodamine B (30 mg/ml) dissolved in either water, 5% sucrose in water, or 50% glycerol in water, are introduced on top of the corresponding agarose gels (2-4). The agarose tubes are inverted after 10 minutes, by which time the dye solution has completely penetrated the gel. The distance traveled by dye front which runs as a clearly, demarcated concave minuscus is measured with time in all tubes. The convective dispersion of dye into the solution above the diffusion front was visible after 1 hour in tube 1 (no agarose) but was absent for at least 9 hours in tube 2 and at least 95 hours in tubes 3 and 4 (Table III).

TABLE III

| Convective Dispersion of Dye in Various Media | |
|---|---|
| Solution | Convective Dispersion of Dye |
| Water | + |
| Water + 1% agarose | -b |
| 50% glycerol + 1% agarose | - c |
| 50% agarose + 1% agarose | - c |
| ameasured as visually detectable color above the diffusion front. | |

bnot detectable after 9 hours.

cnot detectable after 95 hours.

## EXAMPLE V

An agarose gel in water is prepared as described in Example IV, except that it contained mannitol peroxide and guaiac instead of rhodamine B. A stool specimen comprising blood cells is smeared or dotted on the gel. A bluish color develops around the smear or dotted site which remains localized.

## EXAMPLE VI:

2.0 ml of the following solutions are introduced into 10mm x 75 mm glass culture tubes:

1. Water
2. 50% (w/v) sucrose:water
3. 50% (w/v) glycerol:water

Fifteen µl of a solution of rhodamine B (30 mg/ml) dissolved in either solution 1,2 or 3 are carefully injected by syringe into the bottom of the tube containing the corresponding solution (1,2 or 3). These are introduced so that no dye is dispersed in the solution above the bottom of the tube. The distance traveled by dye front which runs as a clearly demarcated concave minuscus is measured with time for all tubes. The rate of diffusion of the dye as indicated by the rate of dye front movement is markedly affected by the viscosity of the solution, taking 5 to 6 times as long to travel the same distance in 50% sucrose or 50% glycerol as in water.

## EXAMPLE VII:

## Diffusion of Dye in Glycerol:Water Solutions of Varying Viscosities.

Glycerol in varying concentrations as shown in Table I hereinbelow was dissolved in water containing one percent by weight agarose. The solution was heated to 80° C to dissolve the agarose and 11 ml of each solution were poured into 10mm x 75mm culture tubes. The solutions were permitted to cool to room temperature resulting in the agarose forming a gel. The tubes were equilibrated in a water bath at 30° C and 10 μl of a solution of rhodamine B in water (30mg/ml) was carefully applied to the top of the gel. The dye was permitted to diffuse into the gel and the distance from the origin to the dye front was measured with time. A viscosity of 1.50 mPa·s (cp) significantly retarded the diffusion of the dye relative to water at 30°C.

TABLE IV

| Diffusion of Rhodamine B in Glycerol:water Solutions of Varying Viscosities | | | | | |
| 5 Wt% | solution | viscosity mPa·s (cp)[a] | Distance moved by dye front (c m)[b] | | |
| | | | 3 hr | 8 hr | 24 hr |
| 0 | | .800 | 14.2±.3 | 20±1 | 30±1.5 |
| 10 | glycerol | 1.02 | 12.5±.2 | 20±1 | 30±1.5 |
| 23 | glycerol | 1.50 | 9.5±.2 | 13.5±.5 | 21±1 |
| 51 | glycerol | 4.47 | 7.7±.3 | 10±.3 | 14.0±.3 |
| 80 | glycerol | 33.9 | 3.5±.2 | 5.0±3 | 9.0±.3 |

[a] At 30 ° C from Lange's Handbook of Chemistry, 11th ed. J.A. Dean (ed) McGraw Hill (1973) p. 289.

[b] At 30 ° C. Average of two determinations.

[c] Sucrose was partially crystallized at this concentration.

## EXAMPLE VIII:

### Diffusion of Dye in Sucrose:Water Solutions of Varying Viscosities.

Sucrose in varying concentrations as shown in Table II hereinbelow was dissolved in water containing one percent by weight agarose, the solution was poured into tubes to form a gel, and the gel layered with Rhodamine B as described in EXAMPLE IV. A viscosity of 1.49 mPa·s (cp) significantly retarded the diffusion of the dye relative to water at 30 ° C.

TABLE V

| Diffusion of Rhodamine B Sucrose:water Solutions of Varying Viscosities | | | | | |
| Wt% | solution | viscosity mPa·s (cp)[a] | Distance moved by dye front (c m)[b] | | |
| | | | 3 hr | 8 hr | 24 hr |
| 0 | | 0.800 | 14.2±.3 | 20±1 | 30±1.5 |
| 20 | sucrose | 1.49 | 9.7±.2 | 14.5±.5 | 21±1 |
| 40 | sucrose | 4.38 | 7.5±.2 | 10.5±.3 | 14.7±.3 |
| 60 | sucrosel | 33.9 | 2.0±.1[c] | 2.5±.1[c] | 14.0±.3 |

[a] At 30 ° C from Lange's Handbook of Chemistry, 11th ed. J.A. Dean (ed) McGraw Hill (1973) p. 289.

[b] At 30° C. Average of two determinations.

[c] Sucrose was partially crystallized at this concentration.

## Claims

1.  A method for enhanced detection of the presence of an analyte moiety by limiting the spread of the signal from the point of origin of the signal comprising the steps of:

    (a) fixing to a support either a first analyte moiety or a first analyte-specific moiety;
    (b) forming a complex comprising either:

       (1) said first analyte moiety and a second analyte-specific moiety, or
       (2) said first analyte-specific moiety and a second analyte moiety, wherein each of said second analyte-specific moiety and said first analyte-specific moiety has a signalling moiety capable of generating a signal either directly or indirectly attached thereto;

(c) forming either specific or viscous medium on said support, wherein said specific medium comprises (1) a solution, (2) a polymer network, and (3) a signal precursor, and wherein said viscous medium comprises (1) a solution, (2) a specific component that imparts the desired viscosity to said viscous medium, and (3) a signal precursor; and

(d) generating a signal by means of said signalling moiety.

2. A method for enhanced detection of the presence of an analyte moiety in a sample by limiting the spread of the signal from the point of origin of the signal wherein said analyte moiety is or comprises either a signalling moiety or a signal precursor, comprising the steps of:

(a) forming either a:

(1) specific or viscous medium on a support when said analyte moiety is or comprises a signalling moiety capable of generating a signal directly or indirectly, wherein said specific medium comprises (a) a solution, (b) a network, and (c) a signal precursor, and wherein said viscous medium comprises (a) a solution, (b) a specific component, and (c) a signal precursor, or

(2) indicative or thick medium on a support when said analyte moiety is or comprises a signal precursor, wherein said indicative medium comprises (a) a solution, (b) a polymer network, and (c) a signalling moiety, and wherein said thick medium comprises (a) a solution, (b) a specific component, and (c) a signalling moiety;

(b) contacting one of said media with said analyte moiety; and

(c) generating a signal by means of said signalling moiety.

3. The method of Claim 1 or 2 wherein said viscosity is from 1.5 mPa·s (cp) to 1000 mPa·s (cp).

4. The method of claim 1 or 2 wherein said polymer network is selected from the polymer group consisting of carbohydrates, polysaccharides, starches, polynucleotides, proteins, polypeptides, polyglycols, liposaccharides, dextrans, methylcelluloses, pectins, mucilages, plant gums, celluloses, agaroses, cross-linked agaroses polyacrylarnides, liquid crystals, zeolites, silicates, phosphates, sulfates and aluminates.

5. The method of Claim 1, 2, or 4 wherein in said signal generating step said network or said support interacts with said signal.

6. The method of Claims 1 or 2 wherein said network is produced by a gel.

7. The method of Claims 1 or 3 wherein in said specific medium forming step, said signal precursor is added to said solution to form said medium before layering or pouring said solution onto said support, or after layering or pouring said solution onto said support.

8. The method of Claim 1, 2, or 7 wherein said specific component is selected from the group consisting of sugars, alcohols, glycols, proteins, polypeptides, amino acids, deoxyribonucleic acids, ribonucleic acids, polynucleotides, nucleotides, nucleosides, bases, fatty acids, pectins, mucins, polysaccharides, steroids, flavanoids, alkaloids, terpenes, vitamins, co-enzmyes, and prostaglandins, hydrocarbons, and porphyrins.

9. The method of Claim 1 or 2 wherein said signal is soluble or insoluble in said medium.

10. The method of Claim 1 or 2 wherein said analyte moiety is selected from the group consisting of ligands, receptors, proteins, antibodies, antigens, nucleic acids, polynucleotides, polysaccharides, polypeptides, microorganisms, viruses, phages, bacteria, fungi, algae, allergens, animal cells, plant cells and tumor cells.

11. The method of Claim 1 or 2 wherein said analyte-specific moiety is selected from the group consisting of: microorganisms, ligands, receptors, glycoproteins, proteins, enzymes, enzyme substrates, enzyme inhibitors, antigens, hormones, antibodies, polynucleotides, amino acids, lipids and lectins.

12. The method of Claim 1 or 2 wherein said signalling moiety is selected from the group consisting of enzymes, catalysts, radical generators, and oxidizing agents.

**Patentansprüche**

1. Verfahren zum verbesserten Nachweis des Vorhandenseins einer Analyteinheit durch Begrenzung der Ausbreitung des Signals vom Ausgangspunkt des Signals aus, umfassend die folgenden Schritte:

   (a) Anheften einer ersten Analyteinheit oder einer ersten analytspezifischen Einheit an einen Träger;
   (b) Bildung eines Komplexes, der:

   (1) die erste Analyteinheit und eine zweite analytspezifische Einheit oder
   (2) die erste analytspezifische Einheit und eine zweite Analyteinheit umfasst, wobei sowohl die zweite analytspezifische Einheit als auch die erste analytspezifische Einheit eine direkt oder indirekt gebundene Signaleinheit aufweisen, die fähig ist, ein Signal zu erzeugen,

   (c) Bildung eines spezifischen oder viskosen Mediums auf dem Träger, wobei das spezifische Medium (1) eine Lösung, (2) ein Polymernetzwerk und (3) eine Signalvorstufe umfasst, und wobei das viskose Medium (1) eine Lösung, (2) eine spezifische Komponente, die dem viskosen Medium die gewünschte Viskosität verleiht, und (3) eine Signalvorstufe umfasst; und
   (d) Erzeugen eines Signals mithilfe der Signaleinheit.

2. Verfahren zum verbesserten Nachweis des Vorhandenseins einer Analyteinheit in einer Probe durch Begrenzung der Ausbreitung des Signals vom Ausgangspunkt des Signals aus, wobei die Analyteinheit eine Signaleinheit oder eine Signalvorstufe ist oder umfasst, umfassend die folgenden Schritte:

   (a) Bildung entweder

   (1) eines spezifischen oder viskosen Mediums auf einem Träger, wenn die Analyteinheit eine Signaleinheit ist oder umfasst, die zur direkten oder indirekten Erzeugung eines Signals fähig ist, wobei das spezifische Medium (a) eine Lösung, (b) ein Netzwerk und (c) eine Signalvorstufe umfasst, und wobei das viskose Medium (a) eine Lösung, (b) eine spezifische Komponente und (c) eine Signalvorstufe umfasst, oder
   (2) eines anzeigenden oder dicken Mediums auf einem Träger, wenn die Analyteinheit eine Signalvorstufe ist oder umfasst, wobei das anzeigende Medium (a) eine Lösung, (b) ein Polymernetzwerk und (c) eine Signaleinheit umfasst, und wobei das dicke Medium (a) eine Lösung, (b) eine spezifische Komponente und (c) eine Signaleinheit umfasst;

   (b) Inkontaktbringen der Medien mit der Analyteinheit; und
   (c) Erzeugen eines Signals mithilfe der Signaleinheit.

3. Verfahren nach Anspruch 1 oder 2, wobei die Viskosität zwischen 1,5 mPa·s (cp) und 1000 mPa·s (cp) liegt.

4. Verfahren nach Anspruch 1 oder 2, wobei das Polymernetzwerk ausgewählt ist aus der Polymergruppe, die aus Kohlenhydraten, Polysacchariden, Stärken, Polynucleotiden, Proteinen, Polypeptiden, Polyglycolen, Liposacchariden, Dextranen, Methylcellulosen, Pectinen, Pflanzenschleim, Pflanzengummen, Cellulosen, Agarosen, vernetzten Agarosen, Polyacrylamiden, flüssigen Kristallen, Zeoliten, Silikaten, Phosphaten, Sulfaten und Aluminaten besteht.

5. Verfahren nach Anspruch 1, 2 oder 4, wobei im signalerzeugenden Schritt das Netzwerk oder der Träger mit dem Signal in Wechselwirkung tritt.

6. Verfahren nach Anspruch 1 oder 2, wobei das Netzwerk durch ein Gel produziert wird.

7. Verfahren nach Anspruch 1 oder 3, wobei in dem Schritt zur Erzeugung des spezifischen Mediums die Signalvorstufe zur Erzeugung des Mediums zu dieser Lösung zugefügt wird, bevor die Lösung auf den Träger aufgebracht oder gegossen wird, oder nachdem die Lösung auf den Träger aufgebracht oder gegossen worden ist.

8. Verfahren nach Anspruch 1, 2 oder 7, wobei die spezifische Komponente ausgewählt ist aus der Gruppe Zucker, Alkohole, Glykole, Proteine, Polypeptide, Aminosäuren, Desoxyribonucleinsäuren, Ribonucleinsäuren, Polynucleotide, Nucleotide, Nucleoside, Basen, Fettsäuren, Pectine, Mucine, Polysaccharide, Steroide, Flavanoide, Alkaloide, Terpene, Vitamine, Co-Enzyme, Prostaglandine, Kohlenwasserstoffe und Porphyrine.

**9.** Verfahren nach Anspruch 1 oder 2, wobei das Signal in dem Medium löslich oder unlöslich ist.

**10.** Verfahren nach Anspruch 1 oder 2, wobei die Analyteinheit ausgewählt ist aus der Gruppe Liganden, Rezeptoren, Proteine, Antikörper, Antigene, Nucleinsäuren, Polynucleotide, Polysaccharide, Polypeptide, Mikroorganismen, Viren, Phagen, Bakterien, Pilze, Algen, Allergene, tierische Zellen, pflanzliche Zellen und Tumorzellen.

**11.** Verfahren nach Anspruch 1 oder 2, wobei die analytspezifische Einheit ausgewählt ist aus der Gruppe Mikroorganismen, Liganden, Rezeptoren, Glycoproteine, Proteine, Enzyme, Enzymsubstrate, Enzyminhibitoren, Antigene, Hormone, Antikörper, Polynucleotide, Aminosäuren, Lipide und Lektine.

**12.** Verfahren nach Anspruch 1 oder 2, wobei die Signaleinheit ausgewählt ist aus der Gruppe Enzyme, Katalysatoren, Radikalerzeuger und Oxidationsmittel.

**Revendications**

**1.** Procédé pour améliorer la détection de la présence d'un élément d'analyte en limitant la propagation du signal à partir du point d'origine du signal comprenant les étapes consistant:

(a) à fixer à un support soit un premier élément de l'analyte soit un premier élément spécifique de l'analyte;
(b) à former un complexe comprenant:

(1) soit ledit premier élément d'analyte et un second élément spécifique de l'analyte;
(2) soit ledit premier élément spécifique de l'analyte et un second élément de l'analyte;

dans lequel chacun desdits second élément spécifique de l'analyte et premier élément spécifique de l'analyte ont un élément de signalisation capable de générer un signal soit directement soit indirectement qui leur est attaché;
(c) à former un milieu soit spécifique soit visqueux sur ledit support, avec lequel milieu spécifique qui comprend (1) une solution, (2) un réseau polymère, et (3) un précurseur de signal, et lequel milieu visqueux qui comprend (1) une solution, (2) un composant spécifique qui confère la viscosité désirée au dit milieu visqueux, et (3) un précurseur de signal; et
(d) à générer un signal au moyen dudit élément de signalisation.

**2.** Procédé pour améliorer la détection de la présence d'une fraction d'analyte en limitant la propagation du signal à partir du point d'origine du signal, dans lequel ledit élément de l'analyte est ou comprend soit un élément de signalisation soit un précurseur de signal, comprenant les étapes consistant:

(a) à former:

(1) soit un milieu spécifique ou visqueux sur un support quand ledit élément de l'analyte est ou comprend un élément de signalisation capable de générer un signal directement ou indirectement, avec ledit milieu spécifique qui comprend (a) une solution, (b) un réseau, et (c) un précurseur de signal, et avec ledit milieu visqueux qui comprend (a) une solution, (b) un composant spécifique, et (c) un précurseur de signal, ou
(2) soit un milieu indicateur ou épais sur un support quand ledit élément de l'analyte est ou comprend un précurseur de signal, avec ledit milieu indicateur qui comprend (a) une solution, (b) un réseau polymère, et (c) un élément de signalisation, et avec ledit milieu épais qui comprend (a) une solution, (b) un composant spécifique, et (c) un élément de signalisation;

(b) à mettre en contact un desdits milieux avec ledit élément de l'analyte; et
(c) à générer un signal au moyen dudit élément de signalisation.

**3.** Procédé selon la revendication 1 ou 2, dans lequel ladite viscosité va de 1,5 mPa.s (cp) à 1 000 mPa.s (cp).

**4.** Procédé selon la revendication 1 ou 2, dans lequel ledit réseau polymère est choisi dans le groupe de polymères formé par les carbohydrates, les polysaccharides, les amidons, les polynucléotides, les protéines, les polypeptides, les polyglycols, les liposaccharides, les dextranes, les méthylcelluloses, les pectines, les mucilages, les gommes végétales, les celluloses, les agaroses, les agaroses à liaisons transversales (cross-linked), les polyacrylamides, les cristaux liquides, les zéolites, les silicates, les phosphates, les sulfates et les aluminates.

5. Procédé selon la revendication 1, 2 ou 4, dans lequel dans ladite étape de génération du signal ledit réseau ou ledit support interagit avec ledit signal.

6. Procédé selon la revendication 1 ou 2, dans lequel ledit réseau est produit par un gel.

7. Procédé selon la revendication 1 ou 3, dans lequel dans ladite étape de formation du milieu spécifique, ledit précurseur de signal est ajouté à ladite solution pour former ledit milieu avant de mettre en couches ou de verser ladite solution sur ledit support ou après avoir mis en couches ou versé ladite solution sur ledit support.

8. Procédé selon la revendication 1, 2, ou 7, dans lequel ledit composant spécifique est choisi dans le groupe formé par les sucres, alcools, glycols, protéines, polypeptides, acides aminés, acides désoxyribonucléiques, acides ribonucléiques, polynucléotides, nucléotides, nucléosides, bases, acides gras, pectines, mucines, polysaccharides, stéroïdes, flavonoïdes, alcaloïdes, terpènes, vitamines, co-enzymes, et prostaglandines, hydrocarbones, et porphyrines.

9. Procédé selon la revendication 1 ou 2, dans lequel ledit signal est soluble ou insoluble dans ledit milieu.

10. Procédé selon la revendication 1 ou 2, dans lequel ledit élément de l'analyte est choisi dans le groupe formé par les ligands, récepteurs, protéines, anticorps, antigènes, acides nucléiques, polynucléotides, polysaccharides, polypeptides, micro-organismes, virus, phages, bactéries, champignons, algues, allergènes, cellules animales, cellules végétales et cellules tumorales.

11. Procédé selon la revendication 1 ou 2, dans lequel ledit élément spécifique de l'analyte est choisi dans le groupe formé par: les micro-organismes, ligands, récepteurs, glycoprotéines, protéines, enzymes, substrats d'enzymes, inhibiteurs d'enzymes, antigènes, hormones, anticorps, polynucléotides, acides aminés, lipides et lectines.

12. Procédé selon la revendication 1 ou 2, dans lequel ledit élément de signalisation est choisi dans le groupe formé par les enzymes, les catalyseurs, les générateurs de radicaux, et les agents d'oxydation.